(19) **Europäisches Patentamt**

**European Patent Office**

**Office européen des brevets**

(11) **EP 1 000 623 A1**

(12) **EUROPEAN PATENT APPLICATION**

(43) Date of publication:
**17.05.2000 Bulletin 2000/20**

(51) Int. Cl.$^7$: **A61K 31/55**, A61P 11/14

(21) Application number: **98118372.6**

(22) Date of filing: **29.09.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant:
**Boehringer Ingelheim International GmbH 55216 Ingelheim am Rhein (DE)**

(72) Inventor: **Kamei, Junzo, Dr.**
**Yokohoma Kanagawa 236-0012 (JP)**

(74) Representative:
**Laudien, Dieter, Dr.**
**Boehringer Ingelheim GmbH**
**Abteilung Patente,**
**Postfach 200**
**55216 Ingelheim (DE)**

(54) **Use of epinastine as antitussivum**

(57)    The present invention relates to a new use of use of epinastine in the form of its enantiomers, a mixture of enantiomers or as a racemate epinastine for the preparation of a medicament with antitussive activity or for the preparation of a medicament with an enhancement of the antitussive effects of antitussiva known from the state of the art - in particular of centrally acting antitussiva of the codeine or dihydrocodeine type.

**EP 1 000 623 A1**

**Description**

The present invention relates to a new use of use of epinastine in the form of its enantiomers, a mixture of enantiomers or as a racemate epinastine as antitussivum and for the enhancement of the antitussive effects of antitussiva in particular of centrally acting antitussiva of the codeine or dihydrocodeine type.

The present invention, moreover, is concerned with the use of epinastine as the racemate or in the form of its enantiomers present as free base or as an addition salt with a pharmacologically acceptable acid alone or in combination with a further antitussivum - in particular a codeine or dihydrocodeine derivative - for the production of a medicament with enhanced antitussive effects.

Description of the invention:

In recent years, the abuse of over-the-counter cough syrups which usually contain active ingredients such as dihydrocodeine, methylephedrine, caffeine and chlorpheniramine, has been spreading among adolescents.

Surprisingly it has now been found that epinastine has antitussive effects and does not exhibit the above mentioned side effects.

Moreover, it has now been found that there exists on one hand a positive interaction between dihydrocodeine and epinastine with reference to the antitussive effects.- On the other hand epinastine does not enhance the reinforcing effects of dihydrocodeine. Therefore, epinastine is shown to be a useful constituent that does not enhance the potential for the psychological dependence on opioid-containing antitussive preparations.

Detailed description of the invention:

Surprisingly it was found that epinastine exhibits itself an antitussive activity and the antitussive effect of dihydrocodeine is enhanced by the simultaneous administration of histamine H1 antagonists. This enhacement was observed when dihydrocodeine was combined with not only centrally acting (chlorpheniramine), but also peripherally acting (epinastine), histamine H1 antagonists. Furthermore, the present results demonstrate that epinastine has an antitussive activity as well as it enhances the antitussive effect of dihydrocodeine to the same degree as chlorpheniramine.

In the following experiments, concurrent dosing of dihydrocodeine and chlorpheniramine produced a significant place preference. On the other hand, dihydrocodeine-induced conditioned place preference was not potentiated in combination with epinastine. This result suggests that epinastine did not enhance the ability of dihydrocodeine to cause psychological dependence.

Materials and Methods

Animals

Male ICR mice (6 weeks old; Tokyo Laboratory Animals Science Co., Ltd., Tokyo, Japan), weighing about 30 g, were used. They had free access to food and water in an animal room which was maintained at $22 \pm 1°C$ with a 12-hour light-dark cycle. This study was carried out in accordance with the Declaration of Helsinki and/or with the guide for the care and use of laboratory animals as adopted by the committee on the care and use of laboratory animals of Hoshi University, which is accredited by the Ministry of Education, Science, Sports and Culture, Japan.

Antitussive assay

The cough reflex was induced as previously described [J. Kamei et al., Eur. J. Pharmacol. **249** (1993) 161; [J. Kamei et al., Eur. J. Pharmacol. **260** (1994) 257]. Briefly, animals were exposed to a nebulized solution of capsaicin (30 mmol/l) under conscious and identical conditions using a body plethysmograph. The coughs produced during a 3-min exposure period were counted. Capsaicin was dissolved in 10 % ethanol and 10 % Tween 80 saline solution at a concentration of 30 mg/ml, and the solution was diluted with saline. The mice were exposed for 3 min to capsaicin 30 min before the injection of drugs to determine the frequency of control coughs. The animals were again exposed to capsaicin aerosol for 60 min after p.o. injection of drugs. Each animal was used only once. The number of coughs produced after drug injection (Ct) was compared with the number of control coughs (Cc). The antitussive effect was expressed as the  % inhibition of the number of coughs = $(Cc - Ct)/Cc \times 100$.

Place conditioning

Place conditioning was conducted as previously using a minor modification of an unbaised procedure [J. Kamei et al., Eur. J. Pharmacol. **318** (1996) 251]. The apparatus used was a shuttle box (15 x 30 x 15 cm: w x 1 x h) which was divided into two compartments of equal size. One compartment was white with a textured floor and the other was black with a smooth floor. For conditioning, mice were confined to one compartment after drug injections and to the compartment after saline injections. Conditioning sessions consisted of 6-alternate-day injections of drug or vehicle (saline). Immediately following drug injection, mice were confined to one compartment. Following vehicle injection, they were confined to the other compartment. The treatment compartment and the presentation order of the drugs and vehicle were counterbalanced for each drug dose. Conditioning sessions were 60 min in duration. On day 7, tests of conditioning were performed as follows: the partition separating the two compartments was raised to 7 cm above the floor, and a neutral platform was inserted along the seam separating the compartments. The time spent in each compartment during a 900-s session was then measured in a blinded fashion by an infrared beam sensor (KN-80, Natume, Tokyo, Japan). The position of the mouse was defined by the position of its body. All sessions were conducted under conditions of dim illumination and masking white noise.

Drugs

Dihydrocodeine phosphate (Sankyo Co., Tokyo, Japan), chlorpheniramine maleate (Sigma Co., St. Luis, MO, USA) and epinastine hydrochloride (Boehringer Ingelheim KG, Ingelheim/Rhein, Germany) were dissolved in saline and injected in a volume of 1.0 ml/kg. Dihydrocodeine and histamine H1 antagonists were combined in accordance with the generally consistent ratio of over-the-counter antitussive preparations (dihydrocodeine: histamine H1 antagonist = 3 : 1).

Statistics

The data regarding antitussive effects are presented as means $\pm$ S.E. $ED_{50}$ values for the antitussive effect and 95 % confidence limits (95 % CL) were determined using linear regression techniques. The potency ration was calculated according to a statistical program (Program 11 of the Pharmacological Calculations System) (8). Conditioning scores represent the time spent in the drug paired place minus the time spent in the saline-paired place, and are expressed as mean $\pm$ S.E. The Wilcoxon test was used to determine whether individual doses produced a significant conditioning ($P<0.05$).

### **Results:**

Antitussive effects:

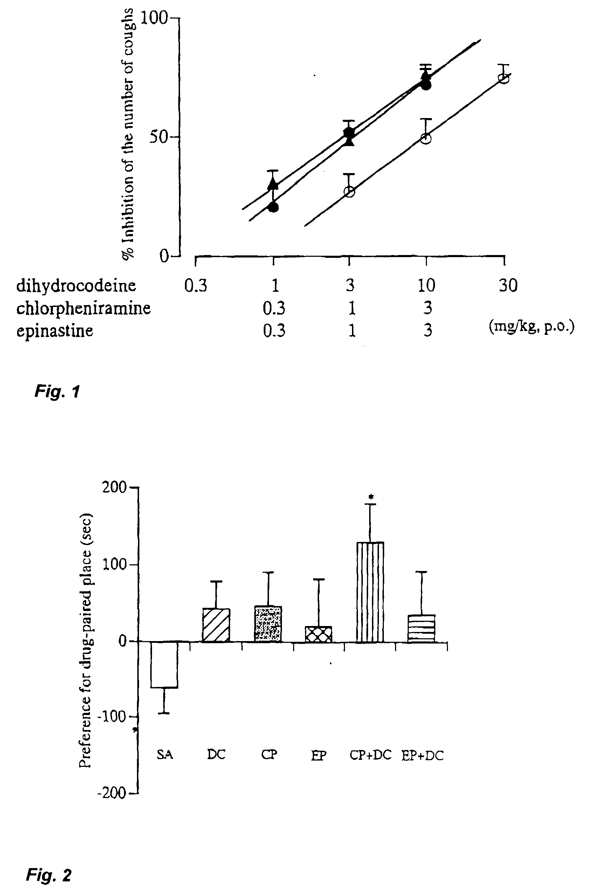

Exposure of control mice to a nebulized solution of capsaicin induced stable coughs (16.5 $\pm$ 0.7 coughs/3 min; n=90). Dihydrocodeine, at doses of 3 - 30 mg/kg, p.o., dose-dependently inhibited the number of capsaicin-induced coughs when the antitussive effect was examined 60 min after administration (Fig. 1). The $ED_{50}$ (95 % CL) of dihydrocodeine was determined to be 9.6 (4.5 - 20.7) mg/kg. The dose-response curve of the antitussive effect of dihydrocodeine was shifted to the left under the co-administration of chlorpheniramine or epinastine. As shown in Table 1, the potentiation of the antitussive effect of dihydrocodeine by chlorpheniramine was similar to that obtained with epinastine.

Table 1

| Effects of chlorpheniramine and epinastine on the antitussive $ED_{50}$ value of dihydrodoceine in mice. | | |
|---|---|---|
| **Treatment** | **$ED_{50}$ (95 % confidence limit) (mg/kg, p.o.)** | **Potency ratio** |
| Dihydrocodeine | 9.6 (4.5 - 20.7) | - |
| Dihydrocodeine + Chlorpheniramine | 3.4 (0.6 - 20.4) | 2.8 (1.6. - 4.8) |
| Dihydrocodeine + Epinastine | 2.8 (0.2 - 11.1) | 3.4 (2.3 - 5.1) |

Place preference:

As shown in Fig. 2, one of the mice that received saline in conditioning sessions exhibited a significant preference for either compartment of the test box. The mean conditioning score was - $60.8 \pm 34.3$ s (n = 8). The place conditionings produced by dihydrocodeine, chlorpheniramine and epinastine are shown in Fig 2. Dihydrocodeine (3 mg/kg, i.p.), chlorpheniramine (1 mg/kg, s.c.) and epinastine (1 mg/kg, s.c.), by themselves, did not produce a remarkable preference or aversion for the drug-associated place; the respective mean conditioning scores were $43.6 \pm 35.8$ s (n=8), $46.7 \pm 44.0$ s (n = 8) and $20.3 \pm 61.8$ s (n = 8). Concurrent dosing of dihydrocodeine (3 mg/kg, i.p.) and chlorpheniramine (1 mg/kg, s.c.) produced a significant place preference, with a mean conditioning score of $130.3 \pm 50.5$ s (n = 8). On the other hand, dihydrocodeine combined with epinastine (1 mg/kg, s.c.) did not produce a significant place preference, with a mean conditioning score of $36.0 \pm 56.0$ s(n = 8).

$ED_{50}$ values for the antitussive effect and 95 % confidence limits (95 % CL) was determined using linear regression techniques. The potency ratio was calculated according to a statistical program (Program 11 of the Pharmacological Calculations System [R.J. Tallarida, and R.B. Murray in: Manual of Pharmacological Calculations with Computer Program. Pp 3 - 166, Raven Press, New York, 1987]).

**The effect of epinastine alone on the capsaicin induced cough reflex**

To further investigate the antitussive effect of epinastine male Hartley guinea-pig - weighing about 350 - were exposed to a nebulized solution of capsaicin (30 $\mu$M) under conscious and identical conditions using a body plethysmograph. The coughs produced during 10-min. exposure period were counted.

Capsaicin was dissolved in 10 % ethanol and 10 % TWEEN 80 saline solution at a concentration of 30 mg/mL, and the solution was diluted with saline. The guinea pigs were exposed for 10 minutes to capsaicin 60 minutes before the administration off epinastine to determine the frequency of control coughs. The animals were again exposed to capsaicin aerosol for 60 minutes after oral administrtion of epinastine. Each animal was used only once. The number of coughs produced after drug administration (Ct) was compared with the number of control coughs (Cc). The antitussive effect was expressed as the %-inhibition of the number of coughs = (Ct-Cc)/Ccx100.

Statistics

The data regarding antitussive effects are presented as means $^{\pm}$S.E.
The Mann-Whitney U-test was used to determine whether individual doses produced are significant (p<0.05)

Results

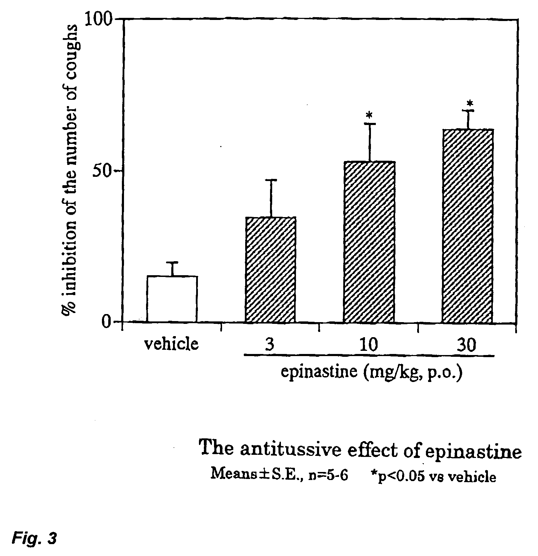

Oral administration of epinastine inhibited the number of capsaicin induced coughs dose-dependently. (3 mg/kg, $34.8 \pm 12.3$ %, n= 5, 10 mg/kg. $53.2 \pm 12,8$ %, n = 6, 30 mg/kg, $64.2 \pm 6.1$ %, n = 6). The results are shown in Fig. 3.

**Figure Legends**

Fig 1 shows the effects of epinastine (closed triangle) and chlorpheniramine (closed circle) on the antitussive effect of dihydrocodeine (open circle: dihydrocodeine alone) in mice. The antitussive effect was assessed 60 min after p.o. administration of dihydrocodeine. Dihydrocodeine and histamine H1 antagonists were combined in accordance with the generally consistent ration of over-the-counter antitussive preparations (dihydrocodeine: histamine H1 antagonist = 3 : 1). Each point represents the mean with S.E. (n= 10).

Fig. 2 shows the effect of chlorpheniramine or epinastine on the place conditioning produced by dihydrocodeine. Each point represents the mean conditioning score $\pm$ S.E. of 8 - 12 mice. SA: saline alone (1 mg/kg, i.p.) group: DC: dihydrocodeine (3 mg/kg, i.p.) group; chlorpheniramine (1 mg/kg, s.c.) group; EP: epinastine (1 mg/kg, s.c.) group; CP+DC: dihydrocodeine (3 mg/kg, i.p.) combined with chlorpheniramine (1 mg/kg, s.c.) group; EP+DC: dihydrocodeine (3 mg/kg, i.p.) combined with epinastine (1 mg/kg, s.c.) group. The asterisks denote significant conditioning (Wilcoxon test: *P<0.05 vs. respective saline alone).

Fig. 3 shows the %-inhibition of the number of coughs depending on various doses of epinastine given orally (3mg/kg, $34.8 \pm 12.3$ %, n= 5, 10 mg/kg. $53.2 \pm 12,8$ %, n = 6, 30 mg/kg, $64.2 \pm 6.1$ %, n = 6).

The following examples of formulations serve for further illustrate the present invention:

Tablets:

1. A tablet contains for example the following ingredients:

| Epinastine | 0.020 parts |
|---|---|
| Dihydrocodeine phosphate | 0.020 parts |
| Stearic acid | 0.010 parts |
| Dextrose | 1.950 parts |
| Total | 2.000 parts |

Preparation:

The substances are mixed together in known manner and the mixture is compressed to from tablets, each weighing 2.000 grams.

**Claims**

1. Use of epinastine in the form of its enantiomers, a mixture of enantiomers or as a racemate, as free base or as an addition salt with a pharmacologically acceptable acid for the preparation of medicament with antitussive activity.

2. Use of epinastine according to claim 1, chracterised in that epinastine in the form of its enantiomers, a mixture of enantiomers or as a racemate, as free base or as an addition addition salt with a pharmacologically acceptable acid is used together with at least one antitussivum for the production of a medicament with enhanced antitussive activity.

3. Use of epinastine according to claim 2, characterised in that the antitussivum is a codeine or a dihydrocodeine derivative.

Fig. 1

Fig. 2

The antitussive effect of epinastine

Means±S.E., n=5-6    *p<0.05 vs vehicle

*Fig. 3*

European Patent Office

**EUROPEAN SEARCH REPORT**

Application Number

EP 98 11 8372

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN vol. 1998, no. 08, 30 June 1998 (1998-06-30) & JP 10 072349 A (TAISHO PHARMACEUT CO LTD), 17 March 1998 (1998-03-17) * abstract * | 1-3 | A61K31/55 A61P11/14 |
| X | PATENT ABSTRACTS OF JAPAN vol. 1998, no. 08, 30 June 1998 (1998-06-30) & JP 10 072348 A (TAISHO PHARMACEUT CO LTD), 17 March 1998 (1998-03-17) * abstract * | 1-3 | |
| X | PATENT ABSTRACTS OF JAPAN vol. 1998, no. 05, 30 April 1998 (1998-04-30) & JP 10 017473 A (TAISHO PHARMACEUT CO LTD), 20 January 1998 (1998-01-20) * abstract * | 1 | |
| Y | MCLEOD R L ET AL: "Antitussive action of antihistamines is independent of sedative and ventilation activity in the guinea pig." PHARMACOLOGY, (1998 AUG) 57 (2) 57-64. , XP000878803 * the whole document * | 1 | TECHNICAL FIELDS SEARCHED (Int.Cl.7)  A61K |
| Y | THE MERK RESEARCH LABORATORIES: "The Merk Index (twelfth edition)" 1996 , MERK & CO., INC. , NJ, (USA) XP002130494 * See epinastine * * page 613, column 1-2 * | 1 | |

-/--

The present search report has been drawn up for all claims

| Place of search | Date of completion of the search | Examiner |
|---|---|---|
| THE HAGUE | 14 February 2000 | Veronese, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

**EUROPEAN SEARCH REPORT**

Application Number

EP 98 11 8372

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| E | PATENT ABSTRACTS OF JAPAN vol. 1999, no. 08, 30 June 1999 (1999-06-30) & JP 11 071281 A (TAISHO PHARMACEUT CO LTD), 16 March 1999 (1999-03-16) & CHEMICAL ABSTRACT 'On line! SERVICE AN=1999:181641 * abstract * | 1 | |
| E | WO 99 32125 A (SCHERING CORP ;DANZIG MELVYN R (US); JENSEN PEDER K (US); MEDEIROS) 1 July 1999 (1999-07-01) * page 1, line 17,18 * * page 5, line 13-20 * * claim 2 * | 1 | |

TECHNICAL FIELDS SEARCHED (Int.Cl.7)

The present search report has been drawn up for all claims

| Place of search | Date of completion of the search | Examiner |
|---|---|---|
| THE HAGUE | 14 February 2000 | Veronese, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 98 11 8372

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2000

| Patent document cited in search report | Publication date | Patent family member(s) | Publication date |
|---|---|---|---|
| JP 10072349 A | 17-03-1998 | NONE | |
| JP 10072348 A | 17-03-1998 | NONE | |
| JP 10017473 A | 20-01-1998 | NONE | |
| JP 11071281 A | 16-03-1999 | NONE | |
| WO 9932125 A | 01-07-1999 | AU 1907199 A | 12-07-1999 |

EPO FORM P0459

For more details about this annex : see Official Journal of the European Patent Office, No. 12/82